(19) Europäisches Patentamt
European Patent Office
Office européen des brevets

(11) Publication number: **0 429 627 B1**

(12) **EUROPEAN PATENT SPECIFICATION**

(45) Date of publication of patent specification: **13.09.95**

(51) Int. Cl.[6]: **C07D 215/56**, A61K 31/47

(21) Application number: **90909805.5**

(22) Date of filing: **31.05.90**

(86) International application number:
**PCT/SE90/00375**

(87) International publication number:
**WO 90/15052 (13.12.90 90/28)**

(54) **DERIVATIVES OF OUINOLINE-3-CARBOXANILIDE.**

(30) Priority: **09.06.89 SE 8902076**

(43) Date of publication of application:
**05.06.91 Bulletin 91/23**

(45) Publication of the grant of the patent:
**13.09.95 Bulletin 95/37**

(84) Designated Contracting States:
**AT BE CH DE DK ES FR GB IT LI LU NL SE**

(56) References cited:
**EP-A- 0 059 698**
**EP-A- 0 165 474**

(73) Proprietor: **Pharmacia AB (reg.number 556131-9608)**

**S-171 97 Stockholm (SE)**

(72) Inventor: **GUNNARSSON, Per, Olov**
**Gartnergatan 4**
**S-252 51 Helsingborg (SE)**
Inventor: **STALHANDSKE, Torbjörn**
**Brännögatan 13**
**S-253 72 Helsingborg (SE)**
Inventor: **STRANDGARDEN, Kerstin**
**Blomsterstigen 3**
**S-260 30 Vallakra (SE)**
Inventor: **LUNDVALL, Karl-Erik**
**Espehögsvägen 69**
**S-262 00 Ängelholm (SE)**
Inventor: **JOHANSSON, Kaj**
**Rektorsgatan 22**
**S-252 36 Helsingborg (SE)**

**EP 0 429 627 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to novel quinoline-3-carboxanilide derivatives, processes for their preparation, pharmaceutical compositions containing them and methods of treatment therewith.

The object of this invention is to provide novel compounds which can be used in therapy of diseases which respond to treatment with immunomodulating agents.

### Prior art

A group of heterocyclic carboxamides which increase the activity of the immune system is disclosed in US pat no 4,547,511. A compound which is included in said patent is roquinimex, also known by the code no LS 2616.

The immunomodulating activity of LS 2616 is described further in ref (2).

### Disclosure of the invention

It has been found that compounds of the general formula I possess useful immunomodulating activity. These compounds, unlike those described in the above mentioned US patent, have no substituents on the nitrogen atom of the quinoline ring.

It is known (3) that N-methyl groups are converted by metabolic reactions to formaldehyde which is a toxic substance. The compounds of the general formula I thus have a lower potential to produce toxic metabolites, which is an advantage especially in individuals with impaired liver function.

The compounds of the general formula I also show higher chemical stability in free form compared with corresponding known compounds cited above. This makes them more useful in certain types of pharmaceutical compositions, e g those intended for transdermal application where it is essential that the active ingredient be in neutral form.

### Summary of the invention

The invention comprises compounds having the general formula

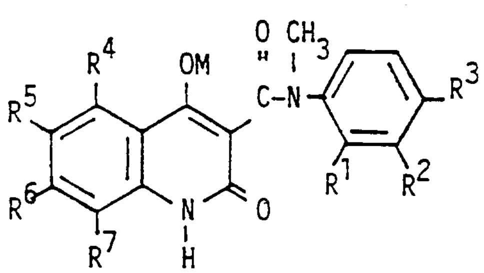

I

wherein $R^1$, $R^2$, $R^3$, $R^4$, $R^5$, $R^6$ and $R^7$ are selected from the group consisting of hydrogen, hydroxy and methoxy. At most one of $R^1$, $R^2$ and $R^3$ and at most one of $R^4$, $R^5$, $R^6$ and $R^7$ is selected from the group consisting of hydroxy and methoxy.

M is selected from the group consisting of hydrogen and pharmaceutically acceptable inorganic or organic cations.

As it is apparent to one skilled in the art the compounds of the general formula I may exist in different tautomeric forms.

### Methods of preparation

The compounds of the general formula I are prepared by conventional methods.

### Method I

Compounds of the general formula I are prepared by reacting a carboxylic acid II or a reactive derivative thereof

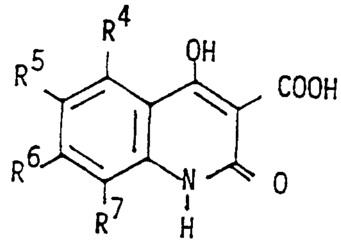

II

with an amine of the general formula III or a reactive derivative thereof

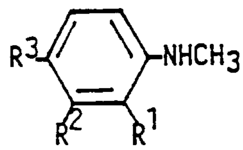

III

Having a reactive derivative of II the reaction may be carried out by mixing the reagents in an inert solvent medium at a temperature between 0° and 200°C depending on the reactivity of the reactive derivative of the carboxylic acid used. As such reactive derivatives the following conventional types may be mentioned: acid chlorides, anhydrides, mixed anhydrides with aliphatic and aromatic sulphonic acids, and reactive derivatives obtained with carbodiimides and similar reagents.

The carboxylic acids II and the reactive derivatives thereof may be prepared by conventional methods as described in (4). The amines III are known compounds.

Method II

A compound of the general formula I wherein at least one of the groups $R^1$-$R^7$ is a hydroxy group and none of $R^1$-$R^7$ is a methoxy group may be prepared by deal kylation of a compound of the general formula having lower alkoxy (preferably methoxy) groups in positions where hydroxy groups are desired.

The dealkylation is preferably carried out with boron tribromide.

The starting materials of the general formula I having methoxy groups in desired positions may be prepared using the type of reaction described in Method I above and in references given there.

Example 1

N,N-dicyklohexylcarbodiimide (2.2 parts) is added to a mixture consisting of 1,2-dihydro-4-hydroxy-2-oxo-quinoline-3-carboxylic acid (2.0 parts), N-metylaniline (1.1 parts) and dry toluene (25 parts) while stirring. The stirring is continued at 110°C for 4 hrs. The reaction mixture is cooled to room temperature and the precipitate formed is filtered off. The precipitate is dissolved at pH 8.2 in 2M sodium hydroxide solution and clarified by filtration to remove the N,N-dicyklohexylurea formed. The solution is acidified with hydrochloric acid solution and the precipitate formed is filtered off. The precipitate is washed with water and methanol and dried to give N-methyl-N-phenyl-1,2-dihydro-4-hydroxy-2-oxo-quinoline-3-carboxamide, m p >300°C (1:1).

In essentially the same manner the following compounds are obtained:

N-methyl-N-(4-methoxyphenyl)-1,2-dihydro-4-hydroxy-2-oxo-quinoline-3-carboxamide, m p >250°C (1:2)

N-methyl-4-phenyl-1,2-dihydro-4-hydroxy-6-methoxy-2-oxo-quinoline-3-carboxamide, m p >250°C (1:3)

N-methyl-N-(4-methoxyphenyl)-1,2-dihydro-4-hydroxy-6-methoxy-2-oxo-quinoline-3-carboxamide, m p >250°C (1:4)

N-methyl-N-(3-methoxyphenyl)-1,2-dihydro-4-hydroxy-7-methoxy-2-oxo-quinoline-3-carboxamide, m p >250°C (1:5).

Example 2

N-methyl-N-(4-methoxyphenyl)-1,2-dihydro-4-hydroxy-2-oxo-quinoline-3-carboxamide (6.5 parts) is suspended in 100 parts of methylene chloride and the mixture obtained is cooled to -60 ° C.

A solution of boron tribromide (20 parts) in methylene chloride (100 parts) is added dropwise while stirring. The mixture is allowed slowly to assume room temperature and is then poured into ice-water. The precipitate obtained is filtered off, washed with water and dried to give N-methyl-N-(4-hydroxyphenyl)-1,2-dihydro-4-hydroxy-2-oxo-quinoline-3-carboxamide, m p 250 ° C (2:1).

In essentially the same manner the following compounds are obtained:

N-methyl-N-phenyl-1,2-dihydro-4,6-dihydroxy-2-oxo-quinoline-3-carboxamide (2:2)
N-methyl-N-phenyl-1,2-dihydro-4,7-dihydroxy-2-oxo-quinoline-3-carboxamide (2:3)
N-methyl-N-(4-hydroxy-phenyl)-1,2-dihydro-4-hydroxy-2-oxo-quinoline-3-carboxamide (2:4)
N-methyl-N-(4-hydroxy-phenyl)-1,2-dihydro-4,6-dihydroxy-2-oxo-quinoline-3-carboxamide (2:5)

Example 3

This example illustrates the effect of the compounds of the general formula I in the oxazolone induced hypersensitivity test in mice. A modification of a method described in (5) was used.

Female mice BALB/c weighing 18-20 g were purchased from Bomholdtgaard Breeding and Research Centre, DH-8680 Ry, Denmark. The animals were randomized in groups of five animals.

Mice were sensitized day 0 by epicutaneous application of 150 µl of absolute ethanol-aceton (3:1) solution containing 3% oxazolone (4-ethoxymethylene-2-phenyloxazol-5-one) on the shaved thorax and abdomen. Seven days after the sensiti zation (day 7) both ears of all mice were challenged on both sides by topical application of 20 µl of 1% oxazolone in peanut oil. Ear thickness was measured prior to 0 hrs and 24 hrs after challenge, using an Oditest spring caliper (Kröplin, Hessen, FRG). The test compounds were administered, suspended in aqueous methocel solution in a volume of 10 mg/kg, by gastric intubation once daily on days 0, 1, 2 and 3. The control animals received the same volume of aqueous methocel solution by gastric intubation.

The results are expressed in percent increase of the ear thickness of the animals treated with the test compounds as compared with the control animals treated with the vehicle.

Table 1

| Preliminary results from the oxazolone induced hypersensitivity test in mice: | | |
|---|---|---|
| Compound | Dose mg/kg p o | Increase of ear thickness, % 24 hrs |
| 1:1 | 4 x 10 | 75.7 |
| 1:2 | 4 x 10 | 79.6 |
| Roquinimex* | 4 x 10 | 47.3 |

* N-methyl-N-phenyl-1,2-dihydro-4-hydroxy-1-methyl-2-oxo-quinoline-3-carboxamide.

The following compounds were also tested in the oxazolone induced hypersensitivity test in mice as described above and produced an increase in ear thickness:

N-methyl-N-(4-methoxyphenyl)-1,2-dihydro-4-hydroxy-2-oxo-quinoline-3-carboxamide (1:2)
N-methyl-4-phenyl-1,2-dihydro-4-hydroxy-6-methoxy-2-oxo-quinoline-3-carboxamide (1:3)
N-methyl-N-(-4-methoxyphenyl)-1,2-dihydro-4-hydroxy-6-methoxy-2-oxo-quinoline-3-carboxamide (1:4)
N-methyl-N-(3-methoxyphenyl)-1,2-dihydro-4-hydroxy-7-methoxy-2-oxo-quinoline-3-carboxamide (1:5).
N-methyl-N-phenyl-1,2-dihydro-4,6-dihydroxy-2-oxo-quinoline-3-carbox amide (2:2)
N-methyl-N-phenyl-1,2-dihydro-4,7-dihydroxy-2-oxo-quinoline-3-carbox amide (2:3)
N-methyl-N-(4-hydroxy-phenyl)-1,2-dihydro-4-hydroxy-2-oxo-quinoline-3-carboxamide (2:4)
N-methyl-N-(4-hydroxy-phenyl)-1,2-dihydro-4,6-dihydroxy-2-oxo-quino line-3-carboxamide (2:5)

References

(1) US pat no 4,547,511
(2) A. Tarkowski et al, Arthritis Rheum. 29 1405 (1986)

(3) Manfred E. Wolff (Ed.), Burger's Medicinal Chemistry, John Wiley & Sons, New York 1980 vol. 1 p. 146 ff
(4) G. Jones (Ed.), Quinolines part 1, John Wiley & Sons (1977) p. 93-318
(5) U. Bicker et al, Journal of Immunopharmacology 6 (1-2) 57 (1984).

## Claims

**Claims for the following Contracting States : AT, BE, CH, DE, DK, FR, GB, IT, LI, LU, NL, SE**

1. Novel compounds having the general formula

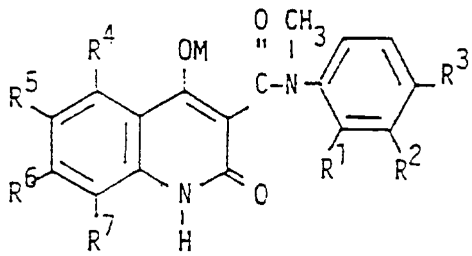

I

wherein $R^1$, $R^2$, $R^3$, $R^4$, $R^5$, $R^6$ and $R^7$ are selected from the group consisting of hydrogen, hydroxy and methoxy. At most one of $R^1$, $R^2$ and $R^3$ and at most one of $R^4$, $R^5$, $R^6$ and $R^7$ is selected from the group consisting of hydroxy and methoxy;
M is selected from the group consisting of hydrogen and pharmaceutically acceptable inorganic or organic cations or tautomeric forms thereof.

2. A compound according to Claim 1, wherein said compound is N-methyl-N-phenyl-1,2-dihydro-4-hydroxy-2-oxo-quinoline-3-carboxamide.

3. A compound according to Claim 1, wherein said compound is N-methyl-N-(4-methoxyphenyl)-1,2-dihydro-4-hydroxy-2-oxo-quinoline-3-carboxamide.

4. A compound according to Claim 1 wherein said compound is N-methyl-4-phenyl-1,2-dihydro-4-hydroxy-6-methoxy-2-oxo-quinoline-3-carboxamide

5. A compound according to Claim 1 wherein said compound is N-methyl-N-(4-methoxyphenyl)-1,2-dihydro-4-hydroxy-6-methoxy-2-oxo-quinoline-3-carboxamide.

6. A compound according to Claim 1 wherein said compound is N-methyl-N-(3-methoxyphenyl)-1,2-dihydro-4-hydroxy-7-methoxy-2-oxo-quinoline-3-carboxamide.

7. A compound according to Claim 1, wherein said compound is N-methyl-N-(4-hydroxyphenyl)-1,2-dihydro-4-hydroxy-2-oxo-quinoline-3-carboxamide.

8. A compound according to Claim 1, wherein said compound is N-methyl-N-phenyl-1,2-dihydro-4,6-dihydroxy-2-oxo-quinoline-3-carboxamide.

9. A compound according to Claim 1 wherein said compound is N-methyl-N-phenyl-1,2-dihydro-4,7-dihydroxy-2-oxo-quinoline-3-carboxamide.

10. A compound according to Claim 1 wherein said compound is N-methyl-N-(4-hydroxy-phenyl)-1,2-dihydro-4-hydroxy-2-oxo-quinoline-3-carboxamide.

11. A compound according to Claim 1 wherein said compound is N-methyl-N-(4-hydroxy-phenyl)-1,2-dihydro-4,6-dihydroxy-2-oxo-quinoline-3-carboxamide.

12. Pharmaceutical compositions containing as an active ingredient one or more of the compounds having the general formula I, and, if desired other pharmacologically active ingredients.

**13.** Pharmaceutical compositions containing as an active ingredient one or more of the compounds having the general formula I together with a pharmaceutically acceptable carrier, and, if desired, other pharmacologically active ingredients.

**14.** Use of compounds of the general formula I

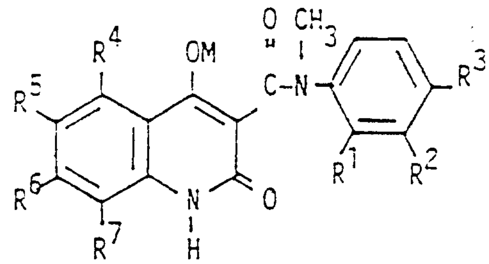

I

wherein $R^1$, $R^2$, $R^3$, $R^4$, $R^5$, $R^6$ and $R^7$ are selected from the group consisting of hydrogen, hydroxy and methoxy; at most one of $R^1$, $R^2$ and $R^3$ and at most one of $R^4$, $R^5$, $R^6$ and $R^7$ is selected from the group consisting of hydroxy and methoxy; M is selected from the group consisting of hydrogen and pharmaceutically acceptable inorganic or organic cations or tautomeric forms thereof, for the manufacture of pharmaceutical compositions for the treatment of diseases which respond to treatment with immunomodulating agents.

**15.** A process for the manufacture of novel compounds having the general formula

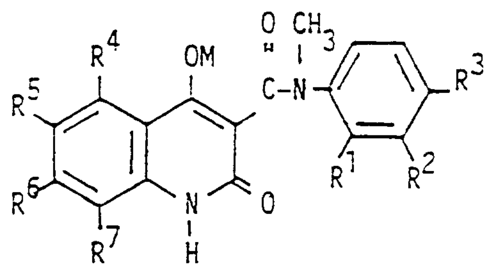

I

wherein $R^1$, $R^2$, $R^3$, $R^4$, $R^5$, $R^6$ and $R^7$ are selected from the group consisting of hydrogen, hydroxy and methoxy; at most one of $R^1$, $R^2$ and $R^3$ and at most one of $R^4$, $R^5$, $R^6$ and $R^7$ is selected from the group consisting of hydroxy and methoxy; M is selected from the group consisting of hydrogen and pharmaceutically acceptable inorganic or organic cations or tautomeric forms thereof, **characterized** by reacting a carboxylic acid with the formula II or a reactive derivative thereof

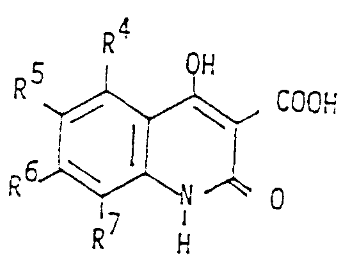

II

with an amine of the general formula III or a reactive derivative thereof

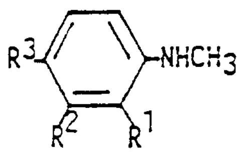

III

whereby $R^1$, $R^2$, $R^3$, $R^4$, $R^5$, $R^6$ and $R^7$ have the aforesaid meaning, and wherein the compounds of the general formula I obtained in the above reactions are converted, if desired, into pharmaceutically acceptable addition salts.

**16.** A process for the manufacture of novel compounds having the general formula

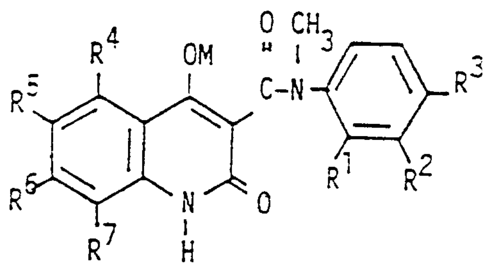

I

wherein $R^1$, $R^2$, $R^3$, $R^4$, $R^5$, $R^6$ and $R^7$ are selected from the group consisting of hydrogen and hydroxy; at least one of $R^1$ - $R^7$ is a hydroxy group; M is selected from the group consisting of hydrogen and pharmaceutically acceptable inorganic or organic cations or tautomeric forms thereof, **characterized** by dealkylation of a compound of the general formula I having lower alkoxy, preferably methoxy, groups in positions where hydroxy groups are desired; and wherein the compounds of the general formula I obtained in the above reactions are converted, if desired, into pharmaceutically acceptable addition salts.

**Claims for the following Contracting State : ES**

**1.** A process for the manufacture of novel compounds having the general formula

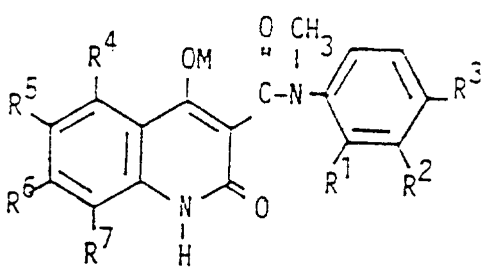

I

wherein $R^1$, $R^2$, $R^3$, $R^4$, $R^5$, $R^6$ and $R^7$ are selected from the group consisting of hydrogen, hydroxy and methoxy; at most one of $R^1$, $R^2$ and $R^3$ and at most one of $R^4$, $R^5$, $R^6$ and $R^7$ is selected from the group consisting of hydroxy and methoxy; M is selected from the group consisting of hydrogen and pharmaceutically acceptable inorganic or organic cations or tautomeric forms thereof, **characterized** by reacting a carboxylic acid with the formula II or a reactive derivative thereof

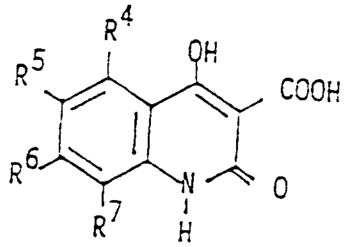

II

with an amine of the general formula III or a reactive derivative thereof

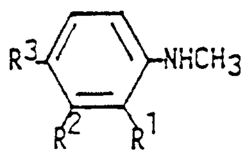

III

whereby $R^1$, $R^2$, $R^3$, $R^4$, $R^5$, $R^6$ and $R^7$ have the aforesaid meaning, and wherein the compounds of the general formula I obtained in the above reactions are converted, if desired, into pharmaceutically acceptable addition salts.

**2.** A process for the manufacture of novel compounds having the general formula

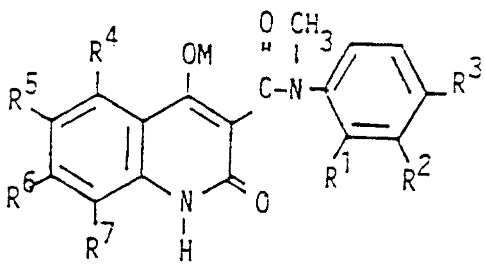

I

wherein $R^1$, $R^2$, $R^3$, $R^4$, $R^5$, $R^6$ and $R^7$ are selected from the group consisting of hydrogen and hydroxy; at least one of $R^1$ - $R^7$ is a hydroxy group; M is selected from the group consisting of hydrogen and pharmaceutically acceptable inorganic or organic cations or tautomeric forms thereof, **characterized** by dealkylation of a compound of the general formula I having lower alkoxy, preferably methoxy, groups in positions where hydroxy groups are desired; and wherein the compounds of the general formula I obtained in the above reactions are converted, if desired, into pharmaceutically acceptable addition salts.

**Patentansprüche**

**Patentansprüche für folgende Vertragsstaaten : AT, BE, CH, DE, DK, FR, GB, IT, LI, LU, NL, SE**

**1.** Neue Verbindung mit der allgemeinen Formel

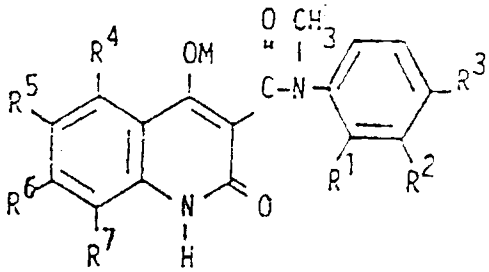

I

worin $R^1$, $R^2$, $R^3$, $R^4$, $R^5$, $R^6$ und $R^7$ aus der Gruppe bestehend aus Wasserstoff, Hydroxy und Methoxy ausgewählt sind. Höchstens eines aus $R^1$, $R^2$ und $R^3$ und höchstens eines aus $R^4$, $R^5$, $R^6$ und $R^7$ ist aus der Gruppe bestehend aus Hydroxy und Methoxy ausgewählt;
M ist aus der Gruppe bestehend aus Wasserstoff und pharmazeutisch zulässigen, anorganischen oder organischen Kationen oder tautomeren Formen hiervon ausgewählt.

**2.** Eine. Verbindung nach Anspruch 1, worin diese Verbindung N-Methyl-N-phenyl-1,2-dihydro-4-hydroxv-2-oxochinoiin-3-carboxamid ist.

**3.** Eine Verbindung nach Anspruch 1, worin diese Verbindung N-Methyl-N-(4-methoxyphenyl)-1,2-dihydro-4-hydroxy-2-oxo-chinolin-3-carboxamid ist.

**4.** Eine Verbindung nach Anspruch 1, worin diese Verbindung N-Methyl-4-phenyl-1,2-dihydro-4-hydroxy-6-methoxy-2-oxo-chinolin-3-carboxamid ist.

**5.** Eine Verbindung nach Anspruch 1, worin diese Verbindung N-Methyl-N-(4-methoxyphenyl)-1,2-dihydro-4-hydroxy-6-methoxy-2-oxochinolin-3-carboxamid ist.

**6.** Eine Verbindung nach Anspruch 1, worin diese Verbindung N-Methyl-N-(3-methoxyphenyl)-1,2-dihydro-4-hydroxy-7-methoxy-2-oxochinolin-3-carboxamid ist.

**7.** Eine Verbindung nach Anspruch 1, worin diese Verbindung N-Methyl-N-(4-hydroxyphenyl)-1,2-dihydro-4-hydroxy-2-oxo-chinolin-3-carboxamid ist.

**8.** Eine Verbindung nach Anspruch 1, worin diese Verbindung N-Methyl-N-phenyl-1,2-dihydro-4,6-dihydroxy-2-oxochinolin-3-carboxamid ist.

**9.** Eine Verbindung nach Anspruch 1, worin diese Verbindung N-Methyl-N-phenyl-1,2-dihydro-4,7-dihydroxy-2-oxochinolin-3-carboxamid ist.

**10.** Eine Verbindung nach Anspruch 1, worin diese Verbindung N-Methyl-N-(4-hydroypheny-1,2-dihydro-4-hydroxy-2-oxo-chinolin-3-carboxamid ist.

**11.** Eine Verbindung nach Anspruch 1, worin diese Verbindung N-Methyl-N-(4-hydroxyphenyl)-1,2-dihydro-4,6-dihydroxy-2-oxochinolin-3-carboxamid ist.

**12.** Pharmazeutische Zusammensetzungen, welche als Wirkstoff eine oder mehrere Verbindungen mit der allgemeinen. Formel I und , falls erwünscht, andere pharmakologish aktive Inhaltsstoffe enthalten.

**13.** Pharmazeutische Zusammensetzungen, welche als Wirkstoff eine oder mehrere Verbindungen mit der allgemeinen Formel I zusammen mit einem pharmazeutisch zulässigen Träger und, falls erwünscht, anderen pharmakologisch aktiven Inhaltsstoffen enthalten.

**14.** Verwendung von Verbindungen der allgemeinen Formel I

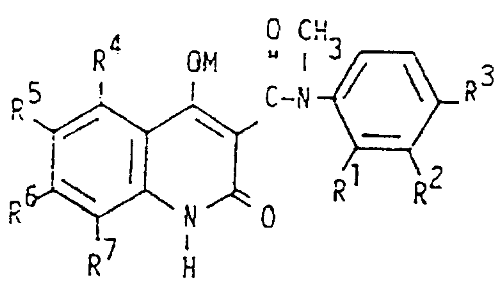

I

worin $R^1$, $R^2$, $R^3$, $R^4$, $R^5$, $R^6$ und $R^7$ aus der Gruppe bestehend aus Wasserstoff, Hydroxy und Methoxy ausgewählt sind; höchstens eines aus $R^1$, $R^2$ und $R^3$ und höchstens eines aus $R^4$, $R^5$, $R^6$ und $R^7$ aus

der Gruppe bestehend aus Hydroxy und Methoxy ausgewählt ist; M aus der Gruppe bestehend aus Wasserstoff und pharmazeutisch zulässigen, anorganischen oder organischen Kationen oder tautomeren Formen hiervon ausgewählt ist, zur Herstellung von Pharmazeutischen Zusammensetzungen zur Behandlung von Erkrankungen, welche auf die Behandlung mit immunmodulierenden Mitteln ansprechen.

**15.** Ein Verfahren zur Herstellung neuer Verbindungen mit der allgemeinen Formel

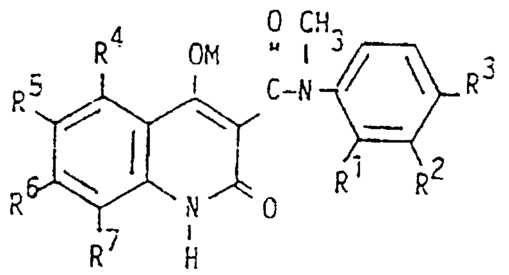

I

worin $R^1$, $R^2$, $R^3$, $R^4$, $R^5$, $R^6$ und $R^7$ aus der Gruppe bestehend aus Wasserstoff, Hydroxy und Methoxy ausgewählt sind; höchstens eines aus $R^1$, $R^2$ und $R^3$ und höchstens eines aus $R^4$, $R^5$, $R^6$ und $R^7$ aus der Gruppe bestehend aus Hydroxy und Methoxy ausgewählt ist; M aus der Gruppe bestehend aus Wasserstoff und pharmazeutisch zulässigen, anorganischen oder organischen Kationen oder tautomeren Formen hiervon ausgewählt ist, dadurch gekennzeichnet, daß man eine Carbonsäure mit der Formel II oder ein reaktives Derivat hiervon

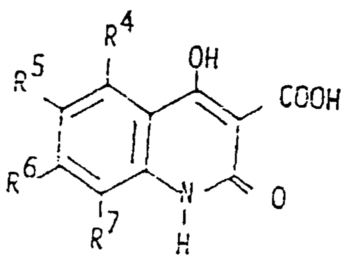

II

mit einem Amin der allgemeinen Formel III oder einem reaktiven Derivat hiervon

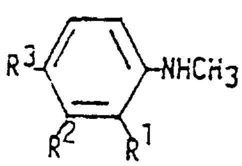

III

umsetzt, worin $R^1$, $R^2$, $R^3$, $R^4$, $R^5$, $R^6$ und $R^7$ die zuvorqenannte Bedeutung haben und worin die in den obigen Reaktionen erhaltenen Verbindungen der allgemeinen Formel I, falls erwünscht, in pharmazeutisch zulässige Additionssalze umgewandelt werden.

**16.** Ein Verfahren zur Herstellung neuer Verbindungen mit der allgemeinen Formel

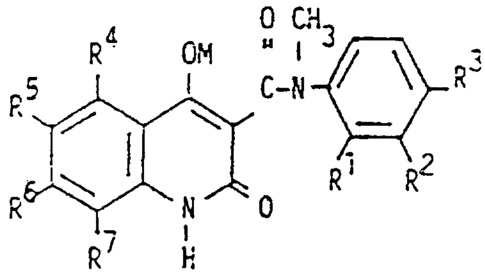

I

worin $R^1$, $R^2$, $R^3$, $R^4$, $R^5$, $R^6$ und $R^7$ aus der Gruppe bestehend aus Wasserstoff und Hydroxy ausgewählt sind; zumindest eines aus $R^1$ - $R^7$ eine Hydroxygruppe ist; M aus der Gruppe bestehend aus Wasserstoff und pharmazeutisch zulässigen, anorganischen oder organischen Kationen oder tautomeren Formen hiervon ausgewählt ist, dadurch gekennzeichnet, daß man eine Verbindung der allgemeinen Formel I mit niedrig Alkoxygruppen, vorzugsweise Methoxygruppen, in Stellungen, wo Hydroxygruppen erwünscht sind, dealkyliert; und worin die in den obigen Reaktionen erhaltenen Verbindungen der allgemeinen Formel I, falls erwünscht, in pharmazeutisch zulässige Additionssalze umgewandelt werden.

**Patentansprüch für folgenden Vertragsstaat : ES**

**1.** Ein Verfahren zur Herstellung neuer Verbindungen mit der allgemeinen Formel

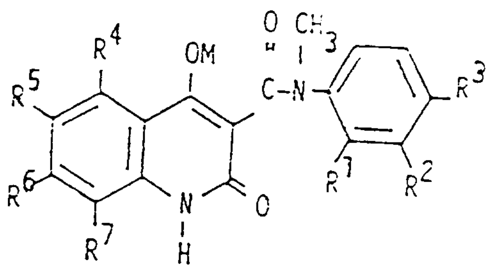

I

worin $R^1$, $R^2$, $R^3$, $R^4$, $R^5$, $R^6$ und $R^7$ aus der Gruppe bestehend aus Wasserstoff, Hydroxy und Methoxy ausgewählt sind; höchstens eines aus $R^1$, $R^2$ und $R^3$ und höchstens eines aus $R^4$, $R^5$, $R^6$ und $R^7$ aus der Gruppe bestehend aus Hydroxy und Methoxy ausgewählt ist; M aus der Gruppe bestehend aus Wasserstoff und pharmazeutisch zulässigen, anorganischen oder organischen Kationen oder tautomeren Formen hiervon ausgewählt ist, dadurch gekennzeichnet, daß man eine Carbonsäure mit der Formel II oder ein reaktives Derivat hiervon

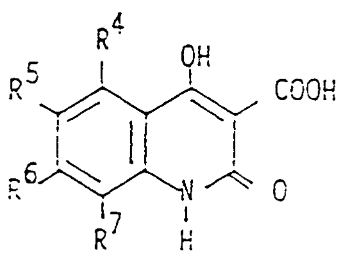

II

mit einem Amin der allgemeinen Formel III oder einem reaktiven Derivat hiervon

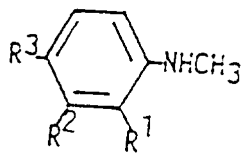

III

umsetzt, worin $R^1$, $R^2$, $R^3$, $R^4$, $R^5$, $R^6$ und $R^7$ die zuvorgenannte Bedeutung haben und worin die in den obigen Reaktionen erhaltenen Verbindungen der allgemeinen Formel I, falls erwünscht, in pharmazeutisch zulässige Additionssalze umgewandelt werden.

**2.** Ein Verfahren zur Herstellung neuer Verbindungen mit der allgemeinen Formel

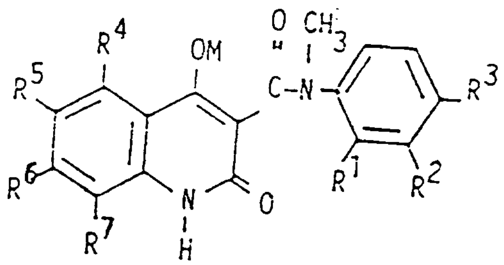

I

worin $R^1$, $R^2$, $R^3$, $R^4$, $R^5$, $R^6$ und $R^7$ aus der Gruppe bestehend aus Wasserstoff und Hydroxy ausgewählt sind; zumindest eines aus $R^1$ - $R^7$ eine Hydroxygruppe ist; M aus der Gruppe bestehend aus Wasserstoff und pharmazeutisch zulässigen, anorganischen oder organischen Kationen oder tautomeren Formen hiervon ausgewählt ist, dadurch gekennzeichnet, daß man eine Verbindung der allgemeinen Formel I mit niedrig Alkoxygruppen, vorzugsweise Methoxygruppen, in Stellungen, wo Hydroxygruppen erwünscht sind, dealkyliert; und worin die in den obigen Reaktionen erhaltenen Verbindungen der allgemeinen Formel I, falls erwünscht, in pharmazeutisch zulässige Additionssalze umgewandelt werden.

**Revendications**
**Revendications pour les Etats contractants suivants : AT, BE, CH, DE, DK, FR, GB, IT, LI, LU, NL, SE**

**1.** Nouveaux composés répondant à la formule générale

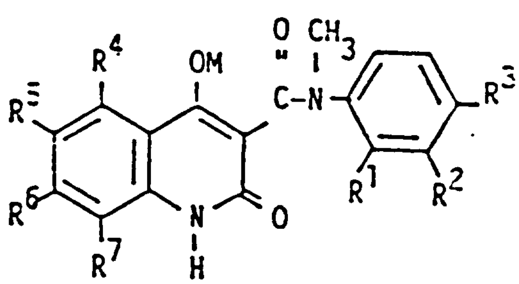

I

dans laquelle $R^1$, $R^2$, $R^3$, $R^4$, $R^5$, $R^6$ et $R^7$ sont choisis dans le groupe constitué par hydrogène, hydroxy et méthoxy ; au plus un de $R^1$, $R^2$ et $R^3$ et au plus un de $R^4$, $R^5$, $R^6$ et $R^7$ est choisi dans le groupe constitué par hydroxy et méthoxy ;

M est choisi dans le groupe constitue par l'hydrogène et les cations minéraux ou organiques pharmaceutiquement acceptables ; ou leurs formes tautomères.

**2.** Composé selon la revendication 1, dans lequel le composé est le N-méthyl-N-phényl-1,2-dihydro-4-hydroxy-2-oxo-quinoline-3-carboxamide.

**3.** Composé selon la revendication 1, dans lequel le composé est le N-méthyl-N-(4-méthoxyphényl)-1,2-dihydro-4-hydroxy-2-oxo-quinoline-3-carboxamide.

**4.** Composé selon la revendication 1, dans lequel le composé est le N-méthyl-4-phényl-1,2-dihydro-4-hydroxy-6-méthoxy-2-oxo-quinoline-3-carboxamide.

**5.** Composé selon la revendication 1, dans lequel le composé est le N-méthyl-N-(4-méthoxyphényl)-1,2-dihydro-4-hydroxy-6-méthoxy-2-oxo-quinoline-3-carboxamide.

**6.** Composé selon la revendication 1, dans lequel le composé est le N-méthyl-N-(3-méthoxyphényl)-1,2-dihydro-4-hydroxy-7-méthoxy-2-oxo-quinoline-3-carboxamide.

**7.** Composé selon la revendication 1, dans lequel le composé est le N-méthyl-N-(4-hydroxyphényl)-1,2-dihydro-4-hydroxy-2-oxo-quinoline-3-carboxamide.

**8.** Composé selon la revendication 1, dans lequel le composé est le N-méthyl-N-phényl-1,2-dihydro-4,6-dihydroxy-2-oxo-quinoline-3-carboxamide.

**9.** Composé selon la revendication 1, dans lequel le composé est le N-méthyl-N-phényl-1,2-dihydro-4,7-dihydroxy-2-oxo-quinoline-3-carboxamide.

**10.** Composé selon la revendication 1, dans lequel le composé est le N-méthyl-N-(4-hydroxy-phényl)-1,2-dihydro-4-hydroxy-2-oxo-quinoline-3-carboxamide.

**11.** Composé selon la revendication 1, dans lequel le composé est le N-méthyl-N-(4-hydroxy-phényl)-1,2-dihydro-4,6-dihydroxy-2-oxo-quinoline-3-carboxamide.

**12.** Compositions pharmaceutiques contenant comme principe actif un ou plusieurs des composés ayant la formule générale I et, si on le souhaite, d'autres principes pharmacologiquement actifs.

**13.** Compositions pharmaceutiques contenant comme principe actif un ou plusieurs des composés ayant la formule générale I conjointement avec un véhicule pharmaceutiquement acceptable et, si on le souhaite, d'autres principes pharmacologiquement actifs.

**14.** Utilisation des composés de la formule générale I

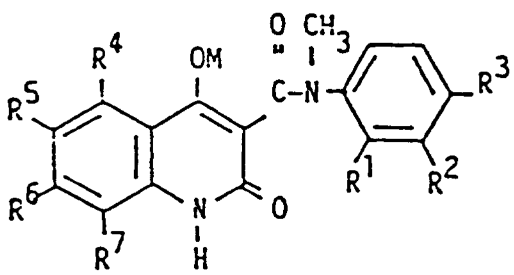

I

dans laquelle $R^1$, $R^2$, $R^3$, $R^4$, $R^5$, $R^6$ et $R^7$ sont choisis dans le groupe constitué par hydrogène, hydroxy et méthoxy ; au plus un de $R^1$, $R^2$ et $R^3$ et au plus un de $R^4$, $R^5$, $R^6$ et $R^7$ est choisi dans le groupe constitué par hydroxy et méthoxy ; M est choisi dans le groupe constitué par l'hydrogène et les cations minéraux ou organiques pharmaceutiquement acceptables ou leurs formes tautomères, pour la fabrication de compositions pharmaceutiques destinées au traitement d'affections qui réagissent au traitement avec des agents immunomodulateurs.

**15.** Procédé pour la fabrication de nouveaux composés de formule générale

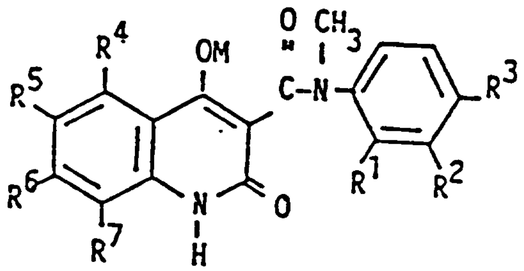

I

dans laquelle $R^1$, $R^2$, $R^3$, $R^4$, $R^5$, $R^6$ et $R^7$ sont choisis dans le groupe constitué par hydrogène, hydroxy et méthoxy ; au plus un de $R^1$, $R^2$ et $R^3$ et au plus un de $R^4$, $R^5$, $R^6$ et $R^7$ est choisi dans le groupe constitué par hydroxy et méthoxy ; M est choisi dans le groupe constitué par l'hydrogène et les cations minéraux ou organiques pharmaceutiquement acceptables ou leurs formes tautomères, caractérisé par la mise en réaction d'un acide carboxylique de formule II ou de son dérivé réactif

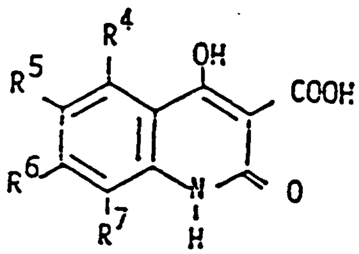

II

avec une amine de formule générale III ou son dérivé réactif

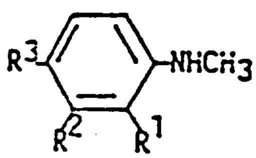

III

moyennant quoi $R^1$, $R^2$, $R^3$, $R^4$, $R^5$, $R^6$ et $R^7$ ont la signification ci-dessus et dans laquelle les composés de la formule générale I obtenus dans les réactions ci-dessus sont convertis, si on le souhaite, en sels d'addition pharmaceutiquement acceptables.

**16.** Procédé pour la fabrication de nouveaux composés de formule générale

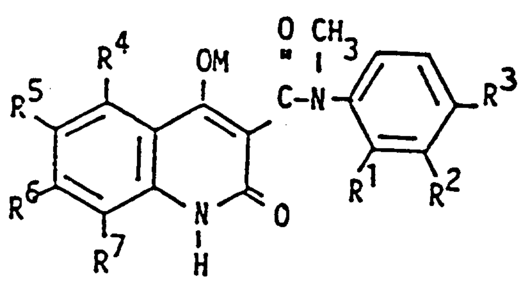

I

dans laquelle $R^1$, $R^2$, $R^3$, $R^4$, $R^5$, $R^6$ et $R^7$ sont choisis dans le groupe constitué par hydrogène et hydroxy ; au moins un de $R^1$ - $R^7$ est un groupe hydroxy ; M est choisi dans le groupe constitué par hydrogène et les cations minéraux ou organiques pharmaceutiquement acceptables ou leurs formes tautomères, caractérisé par la désalkylation d'un composé de formule générale I ayant des groupes

alcoxy inférieurs, de préférence méthoxy, dans des positions où les groupes hydroxy sont souhaités ; et dans lequel les composés de la formule I obtenus dans les réactions ci-dessus sont convertis, si on le souhaite, en sels d'addition pharmaceutiquement acceptables.

**Revendication pou l'Etat contractant suivant : ES**

**1.** Procédé pour la préparation de nouveaux composés ayant la formule générale

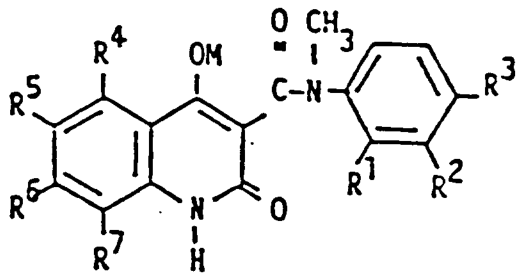

I

dans laquelle $R^1$, $R^2$, $R^3$, $R^4$, $R^5$, $R^6$ et $R^7$ sont choisis dans le groupe constitué par hydrogène, hydroxy et méthoxy ; au moins l'un de $R^1$, $R^2$ et $R^3$ et au plus l'un de $R^4$, $R^5$, $R^6$ et $R^7$ est choisi dans le groupe constitué par hydroxy et méthoxy; M est choisi dans le groupe constitué par l'hydrogène et les cations minéraux ou organiques pharmaceutiquement acceptables ou leurs formes tautomères, caractérisé par la mise en réaction d'un acide carboxylique avec la formule II ou son dérivé réactif

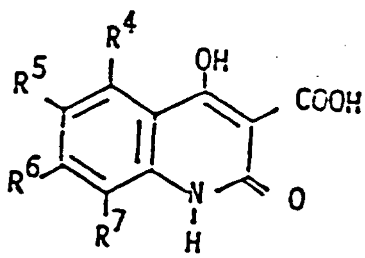

II

avec une amine de formule générale III ou son dérivé réactif

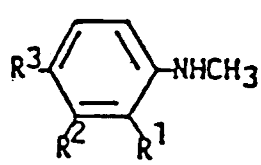

III

moyennant quoi $R^1$, $R^2$, $R^3$, $R^4$, $R^5$, $R^6$ et $R^7$ ont la signification précitée et dans laquelle les composés de la formule générale I obtenus dans les réactions ci-dessus sont convertis, si on le souhaite, en sels d'addition pharmaceutiquement acceptables.

**2.** Procédé pour la fabrication de nouveaux composés de formule générale

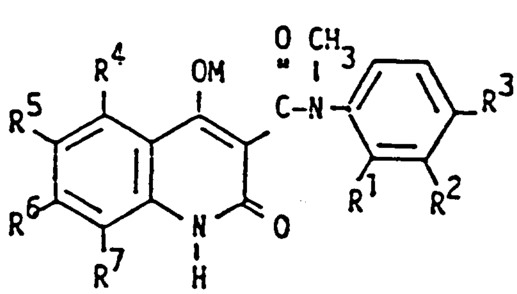

I

dans laquelle $R^1$, $R^2$, $R^3$, $R^4$, $R^5$, $R^6$ et $R^7$ sont choisis dans le groupe constitué par hydrogène et hydroxy ; au moins l'un de $R^1$ - $R^7$ est un groupe hydroxy ; M est choisi dans le groupe constitué par hydrogène et les cations minéraux ou organiques pharmaceutiquement acceptables ou leurs formes tautomères, caractérisé par la désalkylation d'un composé de formule générale I ayant des groupes alcoxy inférieurs, de préférence méthoxy, dans des positions où les groupes hydroxy sont souhaités ; et dans lequel les composés de la formule I obtenus dans les réactions ci-dessus sont convertis, si on le souhaite, en sels d'addition pharmaceutiquement acceptables.